# EUROPÄISCHE PATENTANMELDUNG

(11) **EP 2 072 008 A2**
(43) Veröffentlichungstag der Anmeldung: **24.06.2009**
(21) Anmeldenummer: 08171515.3
(22) Anmeldetag: 12.12.2008
(51) Int. Cl.: A61B 5/024, G06F 3/02

(54) **Vorrichtung zur Bedienung eines elektronischen Multifunktionsgerätes**

(30) Priorität: 19.12.2007 CH 19742007
(71) Anmelder: Hilfiker, Beppo, 4125 Riehen (CH)
(72) Erfinder: Hilfiker, Beppo, 4125 Riehen (CH)
(74) Vertreter: Feldmann, Clarence Paul

(57) **Zusammenfassung**

Die vorliegende Erfindung betrifft eine Bedienungsmechanik eines elektronischen Multifunktionsgerätes (1), welches am Handgelenk tragbar ist und ein Gehäuse mit integrierter Elektronik (4) aufweist. Die Bedienungsmechanik (5) ist in das Gehäuse integriert und weist ein Bedienelement auf, welches eine ein- oder mehrteilige Stellwelle ist. Die Stellwelle quert das Gehäuse teilweise, wobei die Stellwelle parallel zu einer flächigen Ausdehnung des Gehäuses angeordnet ist, rotierbar und linear bewegbar gelagert ist. Mittels einer ersten Bedienkrone (56) und optional mit einer zweiten Bedienkrone (57), ist die Stellwelle linear parallel zur Längsachse und rotativ um die Drehachse/Längsachse bewegbar. Die Bewegung der Stellwelle löst verschiedene Kontakte auf einer Kontaktplatte auf, von welchen elektronische Signale an die Elektronik (4) gesandt werden, wodurch die elektronische Steuerung des Multifunktionsgerätes (1) bewerkstelligt wird.

## Beschreibung

### Technisches Gebiet

Die vorliegende Erfindung beschreibt eine Bedienungsmechanik eines am Handgelenk einer Person tragbaren elektronischen Multifunktionsgerätes, insbesondere eines Pulsmonitors, eines Abspielgerätes für Multimediaanwendungen oder eines Kommunikationsgerätes, wobei das Multifunktionsgerät ein Gehäuse mit einem Gehäuserand aufweist, welches mittels eines Armbandes am Handgelenk lösbar befestigbar ist, wobei innerhalb des Gehäuses die Bedienmechanik und eine integrierte Elektronik angeordnet ist, welche durch eine Mehrzahl elektronischer Kontakte steuerbar ist und mindestens eine mehrzeilige Anzeigeeinheit und eine Sensoreinheit mit einer Mehrzahl von Sensoren für verschiedene Messungen umfasst, wobei mindestens ein erster Mikroprozessor die Datenverarbeitung, die Anzeige von Werten auf der Anzeigeeinheit und die Speicherung von Werten steuert.

### Stand der Technik

In den letzten Jahren ist eine Vielzahl an elektronischen Multifunktionsgeräten, welche portabel am Handgelenk einer Person befestigbar und tragbar sind, auf den Markt gekommen. Dabei werden die mobilen Multifunktionsgeräte, wie beispielsweise Multimedia-Player, Pulsmonitore, mobile Telefone und Navigationsgeräte neben der Messung von physiologischen Parametern, zur Unterhaltung und Kommunikation oder als Navigationshilfe eingesetzt und sind aus dem heutigen Leben nicht mehr wegzudenken.

Der Aufbau dieser elektronischen Multifunktionsgeräte weist ein Gehäuse auf, in dessen Inneren eine Elektronik untergebracht ist, welche das Multifunktionsgerät mit einem oder mehreren Prozessoren steuert. Mit Hilfe der Elektronik werden Daten gemessen, empfangen, ausgewertet und auf einer in das Gehäuse integrierten Anzeigeeinheit angezeigt und können meist in einem Festspeicher gespeichert werden. Zur Steuerung ständig komplexer werdender Multifunktionsgeräte wurde in der Vergangenheit meist eine Mehrzahl an Bedienelementen in Form von in das Gehäuse eindrückbaren Bedientasten benutzt, deren Anzahl auf bis zu fünf und mehr Bedientasten zugenommen hat, wodurch eine Vielzahl an Einstellmöglichkeiten vornehmbar sind.

Da die aktuellen Multifunktionsgeräte aber bereits häufig über weitverzweigte Menübäume verfügen, welche auf einer Anzeigeeinheit abgelesen werden können und durch welche der Benutzer hindurchmanövrieren kann, kommen simple Bedientasten an ihre Grenze.

In Multimedia-Playern ist das Manövrieren durch ein Hauptmenü und diesem untergeordnete Untermenüs mittels Bedienknöpfen vorgesehen. Während ein erster Bedienknopf zum auf und ab Navigieren innerhalb eines Menüs vorgesehen ist, wird ein zweiter Bedienknopf zum Springen in ein Untermenü benötigt. Um in das nächst höhere Menü zu gelangen, kommt ein dritter Bedienknopf zum Einsatz, oder eine Tastenkombination des ersten und zweiten Bedienknopfes muss gleichzeitig gedrückt werden, was für den Anwender zu einer erschwerten Einstellung führt.

Neben den klassischen Bedienknöpfen, welche ein Stück weit in das Gehäuse des Multifunktionsgerätes eingedrückt werden, werden teilweise auch Stellräder, die im Gehäuse drehbar gelagert sind, eingesetzt, welche mit der Fingerspitze um eine Drehachse rotierbar sind und durch deren Bewegung der Benutzer in einem Menü auf und ab manövrieren kann. Ebenso werden Hebel, die um eine im Gehäuse liegende Hebelachse schwenkbar gelagert sind eingesetzt, um das so genannte "Scrollen" durch Menüs und Untermenüs durchführen zu können.

Die Anzahl der Bedientasten wurde im Verlauf der Entwicklung gesteigert und die Art der Bedientasten variiert. So sind heute in einigen Geräten berührungsempfindliche Folientasten zu finden, wobei ein Scrollen durch ein darüber Gleiten eines Fingers über mehrere Folientasten in kurzen Zeitabständen realisiert wird. Es ist aber trotzdem eine Mehrzahl an Bedienelementen nötig, um eine Mehrzahl an Einstellmöglichkeiten bereit zu stellen.

Vorrichtungen des Stands der Technik weisen eine Mehrzahl an Bedienelementen unterschiedlicher Ausführungsformen auf, um das Scrollen in einer Menüstruktur zu realisieren, wobei eine minimale Anzahl an Bedientasten, Stellräder oder Hebeln nötig ist. Die Kombination aus einem, in das Gehäuse eindrückbaren bewegbar gelagerten Rädchens oder Hebels ist ebenfalls bekannt, wodurch ein Scrollen durch das Drehen/Schwenken des Stellrades/Hebels erreicht wird, während ein Sprung in ein Untermenü durch ein Hineindrücken des Stellrades/Hebels realisiert werden kann.

Wenn von einem Hauptmenü ausgehend durch ein tiefer liegendes Untermenü gescrollt werden soll, kann ein Drücken des Stellrades/Hebels in das Gehäuse vorgesehen sein, wobei nach dem Drücken in ein nächst tiefer liegendes Untermenü gesprungen wird. Zum Zurückspringen in das nächst höher liegende Untermenü ist meist ein längeres Drücken vorgesehen, welches durch die Elektronik detektiert wird, so dass durch wiederholte länger dauernde Drückaktionen bis zum höchsten Menü zurück gescrollt werden kann. Mit den bekannten Einstellmöglichkeiten eines rotierbar bewegbaren Stellrades oder Hebels sind die Einstellmöglichkeiten beschränkt und der Benutzer wünscht sich Alternativen.

Da tragbare elektronische Multifunktionsgeräte möglichst klein, leicht und handlich sein sollen, können nur wenige Bedienelemente am Gehäuse angebracht werden, wobei die Grösse der Bedientasten auf die Grösse einer Fingerspitze eines Benutzers angepasst sein muss. Die bisher bekannten elektronischen Multifunktionsgeräte sind teilweise nur noch schwer von Benutzern mit grösseren Händen und Fingern zu benutzen und eine Betätigung während des Tragens von Handschuhen ist oft ausgeschlossen.

### Darstellung der Erfindung

Die vorliegende Erfindung hat sich zur Aufgabe gestellt eine Vorrichtung zur Bedienung eines am Handgelenk tragbaren, uhrenartigen elektronischen Multifunktionsgerätes mit einer mehrzeiligen Anzeigeeinheit zu schaffen, mit welcher durch eine Menüstruktur des Multifunktionsgerätes navigiert werden kann, wobei der Benutzer nur ein einzelnes Bedienelement betätigen muss. Durch die Betätigung des einen Bedienelementes wird ein Scrollen in einem Menü, ein Menüwechsel in weit verzweigte Untermenüs und eine Rückkehr in das Hauptmenü einfach und komfortabel möglich.

Diese Aufgaben und zusätzlich die Bedienung des Multifunktionsgerätes unter Vermeidung des gleichzeitigen Drückens von mehreren Tasten oder die Folgebetätigung von Tastenkombinationen wird durch die vorliegende Vorrichtung gelöst.

Da eine Ausführungsform der erfindungsgemässen Bedienmechanik insbesondere für elektronische Multifunktionsgeräte im Bereich Sport, beispielsweise für Pulsmonitore und Sportuhren Einsatz findet, ist es eine weitere Aufgabe der Erfindung, die Bedienbarkeit für Benutzer mit schweissnassen Händen und Benutzer mit Sporthandschuhen oder bei kalter Witterung mit Winterhandschuhen zu ermöglichen und zu erleichtern, so dass während der Ausübung des Sports Einstellungen einfach und komfortabel durchgeführt werden können, wobei die Hände nicht getrocknet und Handschuhe nicht ausgezogen werden müssen.

### Kurze Beschreibung der Zeichnungen

Die erfindungsgemässe Bedienungsmechanik eines am Handgelenk einer Person tragbaren elektronischen Multifunktionsgerätes wird nachstehend im Zusammenhang mit den Zeichnungen beschrieben, welche eine mögliche Ausführungsform zeigen, wobei
- Figur 1: eine perspektivische Ansicht eines erfindungsgemässen Multifunktionsgerätes mit Stellwelle in Neutralposition zeigt, während
- Figur 2: eine perspektivische Ansicht des Multifunktionsgerätes ohne Gehäusedeckel mit offengelegter Anzeigeeinheit und Bedienungsmechanik zeigt.
- Figur 3: zeigt eine perspektivische Ansicht der Bedienungsmechanik von der Kontaktplattenseite in Neutralposition der Stellwelle, während
- Figur 4: eine perspektivische Ansicht der Bedienungsmechanik von der Basisplatte in Neutralposition der Stellwelle darstellt.

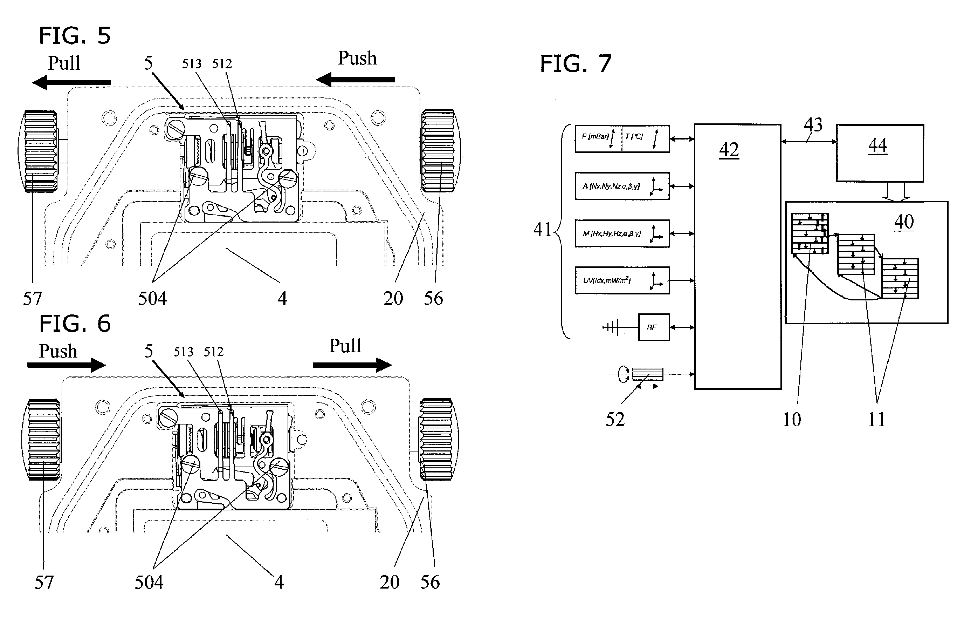

- Figur 5: eine Draufsicht auf die Bedienungsmechanik in der Drückposition der ersten Bedienkrone darstellt.
- Figur 6: zeigt eine Draufsicht auf die Bedienungsmechanik mit zwei Bedienkronen in der Ziehposition der ersten Bedienkrone, während
- Figur 7: eine schematische Darstellung der Elektronik mit Anzeigeeinheit, Sensoreinheit und Mikroprozessor darstellt.
- Figur 8a: zeigt eine Aufsicht auf die Basisplatte, wobei die Stellwelle in Neutralposition ist, während
- Figur 8b: eine Aufsicht auf die Basisplatte zeigt, wobei die Stellwelle in Arretierposition ist.
- Figur 9a: zeigt eine Aufsicht auf die Kontaktplatte gemäss Figur 8a, während
- Figur 9b: eine Aufsicht auf die Kontaktplatte gemäss Figur 8b der Stellwelle in Arretierposition zeigt.
- Figur 10a: zeigt eine perspektivische Ansicht einer möglichen Ausführungsform der Stellwelle und
- Figur 10b: zeigt eine zweidimensionale Aufsicht auf die Stellwelle der Figur 10a, wobei Aussengewinde eingezeichnet sind.
- Figur 11: zeigt eine Aufsicht auf die Kontaktplatte einer möglichen Bedienungsmechanik, wobei
- Figur 11a: einen Schnitt entsprechen der Schnittlinie A-A aus Figur 11 und
- Figur 11b: einen Schnitt entsprechend der Schnittlinie B-B aus Figur 11 zeigt.

### Beschreibung

Im Folgenden wird eine erfindungsgemässe Bedienungsmechanik 5 eines am Handgelenk einer Person tragbaren elektronischen Multifunktionsgerätes 1 am Beispiel eines Pulsmonitors 1 beschrieben. Neben Pulsmonitoren können ebenfalls Multimediaabspielgeräte, sowie Kommunikationsgeräte und weitere Messgeräte mit der hier beschriebenen Bedienungsmechanik ausgestattet werden.

Eine mögliche Ausführungsform eines Pulsmonitors 1 mit der erfindungsgemässen Bedienungsmechanik 5 zur Ermittlung von Parametern, wie Puls, Schrittzahl, Luftdruck, Beschleunigung und Himmelsrichtung umfasst ein Gehäuse 2 mit einem Gehäuserand 20, welches an einem Armband 3 befestigt ist und eine mehrzeilige Anzeigeeinheit 40 aufweist. Auf dieser Anzeigeeinheit 40 lassen sich obige Parameter in verschiedenen Modi darstellen. Ein Benutzer kann zwischen verschiedenen Darstellungsarten auf der Anzeigeeinheit 40 wählen.

Innerhalb des Gehäuses 2 ist eine Elektronik 4 integriert, welche mit der Bedienungsmechanik 5 verbunden ist und durch mindestens einen ersten Mikroprozessor 42 in einer Rechnereinheit gesteuert wird, wobei Daten gemessen, ausgewertet, in einem Speicher abgespeichert und angezeigt werden können. Neben Messwerten wie beispielsweise Pulsfrequenz und Luftdruck, die von Sensoren in einer Sensoreinheit 41 detektierbar und von der Rechnereinheit auslesbar sind, nimmt die Rechnereinheit elektronische Steuerungssignale von der unten detailliert beschriebenen Bedienungsmechanik 5 auf und verarbeitet diese weiter und stellt diese dar.

Die Sensoreinheit 41 kann Temperatursensoren T, Drucksensoren P, Lagesensoren A, Magnetfeldsensoren M, Proximitätssensoren, welche beispielsweise die Nähe zur Haut messen, Berührungssensoren und beispielsweise UV-Sensoren UV umfassen und Mittel zur drahtlosen Kommunikation mit anderen elektronischen Geräten aufweisen, wobei eine RF-Antenne zum Einsatz kommt. Je nach Ausführungsform der Elektronik kann auch ein zweiter Mikroprozessor 44 über eine Schnittstelle 43 mit dem ersten Mikroprozessor 42 in die Elektronik 4 integriert werden, was zu vielfältigen Möglichkeiten führt.

Die Bedienungsmechanik 5 ist in das Gehäuse 2 integriert, mit der Elektronik 4 wirkverbunden und eine einteilige Stellwelle 52 ist als Bedienelement vorgesehen, durch welches die verschiedenen Einstellungen des Pulsmonitors 1 vorgenommen werden können.

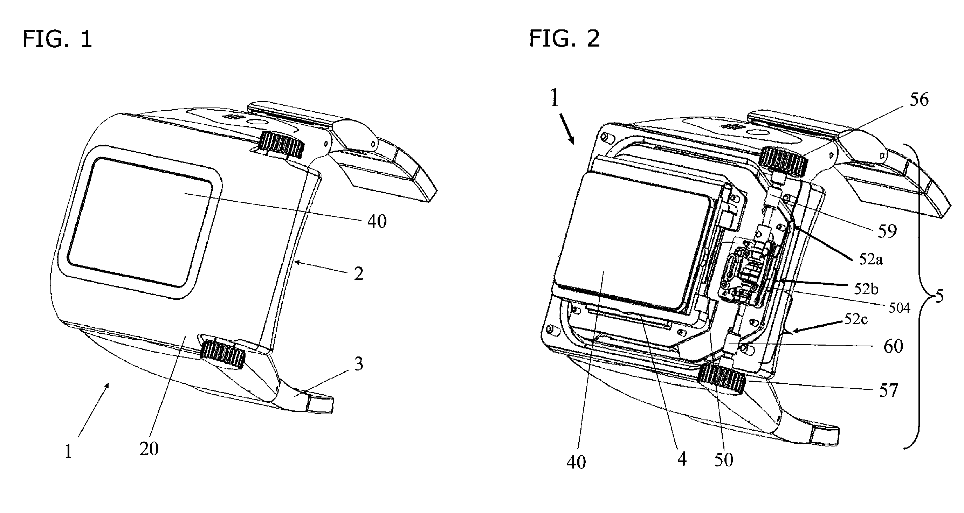

In einer ersten Ausführungsform quert die Stellwelle 52 das Gehäuse 2 teilweise, wobei die Stellwelle 52 parallel zu einer flächigen Ausdehnung des Gehäuses 2 angeordnet ist, rotierbar und linear bewegbar gelagert ist und durch eine Ausnehmung im Gehäuserand 20 aus dem Gehäuse 2 austritt. Die Stellwelle 52 sollte in einer Ebene parallel zur Ebene, in welcher die Anzeigeeinheit 40 liegt, angeordnet sein und könnte demnach auch um 90° verdreht zur in Figur 2 dargestellten Ausrichtung angeordnet sein, oder das Gehäuse 2 kann sogar diagonal von der Stellwelle 52 gequert werden.

Ausserhalb des Gehäuses 2 ist die Stellwelle 52 mit einer ersten Bedienkrone 56 versehen, welche kraft- und/oder formschlüssig mit der Stellwelle 52 koppelbar verbunden ist und eine Riffelung entlang der Mantelfläche der Bedienkrone 56 aufweist, wodurch die erste Bedienkrone 56 griffig und gut bedienbar ist. Die Lagerung der Stellwelle 52 erfolgt in mindestens einem ersten Lager 59, so dass die Stellwelle 52 über eine Basisplatte 50 der Bedienungsmechanik 5 ragt, welche unlösbar im Gehäuse befestigt ist, wobei die Stellwelle 52 in der Art gelagert ist, dass eine lineare Verschiebung parallel zur Längsachse und eine rotative Bewegung um die Drehachse/Längsachse der Stellwelle 52 möglich ist.

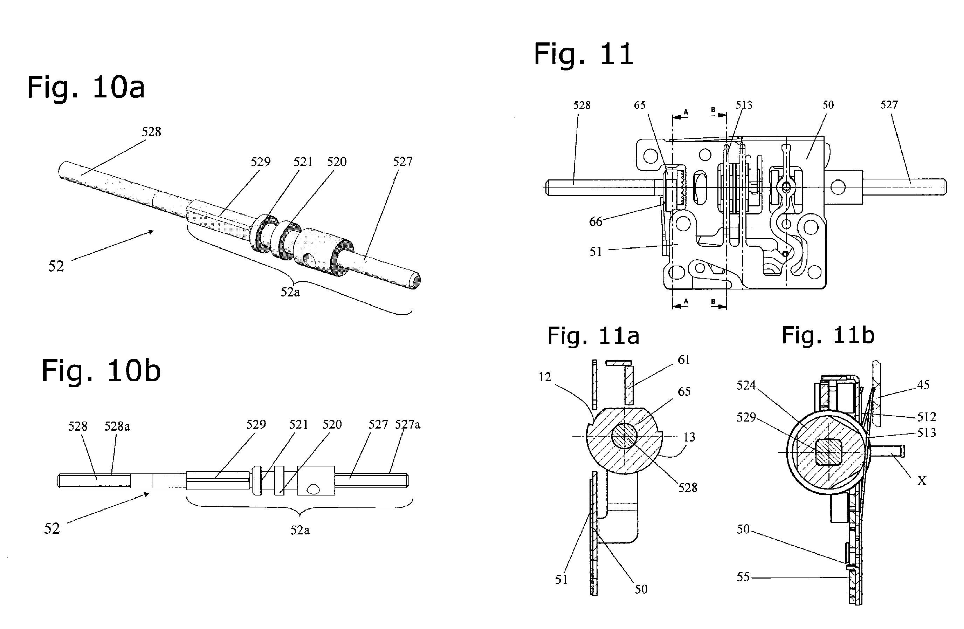

Die hier detailliert beschriebene Stellwelle 52 ist in den Figuren 10a und 10b dargestellt. Die Stellwelle 52 weist einen ersten Kronenzapfen 527 mit einem Gewinde 527a am Anfang der Stellwelle 52 und einen zweiten Kronenzapfen 528 mit einem Gewinde 528a am Ende der Stellwelle 52 auf. Der erste Kronenzapfen 527 wird mit der ersten Bedienkrone 56 lösbar verbunden und ragt aus dem Gehäuse 2 des Multifunktionsgerätes 1 heraus. An den ersten Kronenzapfen 527 schliesst sich eine erste Scheibe 520 und daran eine zweite Scheibe 521 an. Die erste Scheibe 520 und die zweite Scheibe 521 dienen zur Detektion der Linearbewegung der Stellwelle 52, was unten näher erläutert wird. In etwa zentral in der Stellwelle 52 befindet sich ein Steg 529, welcher nicht zylindrisch geformt ist und mit einem Gegenkontaktteil 52b, welcher später näher erläutert wird, wirkverbunden zusammenarbeitet.

Den ersten Kronenzapfen 527, der Steg 529, die erste Scheibe 520 und die zweite Scheibe 521 werden im Folgenden als Schaftteil 52a benannt. Auf dem ersten Kronenzapfen 527 des Schaftteils 52a wird die erste Bedienkrone 56 form- und/oder kraftschlüssig lösbar befestigt. Der dem Schaftteil 52a gegenüberliegende Teil der Stellwelle 52 wird im Folgenden Kupplungsteil 52c genannt und umfasst neben dem zweiten Kronenzapfen 528 mit Gewinde 528a einen Kupplungstrieb 525 mit einem ersten Zahnrad 525a, sowie eine Profilscheibe 65 mit einem zweiten Zahnrad 64. Während der Kupplungstrieb 525 mit daran angeformten oder daran befestigtem ersten Zahnrad mit der Stellwelle 52 mitbewegbar verbunden ist, ist die Profilscheibe 65 mit angeformtem oder befestigtem zweiten Zahnrad 64 unbewegbar mit der Bedienungsmechanik 5 verbunden.

Auf der Basisplatte 50 der Bedienungsmechanik 5 ist der Gegenkontaktteil 52b rotativ bewegbar und linear unbewegbar gelagert. Der Gegenkontaktteil 52b umfasst eine erste Drehrichtungsscheibe 523 und eine zweite Drehrichtungsscheibe 524 auf, welche jeweils Bohrungen mit rechteckigem Querschnitt aufweisen. Die Stellwelle 52 ist auf der Basisplatte 50 derart rotativ und linear bewegbar gelagert, dass der Steg 529 der Stellwelle 52 innerhalb der Bohrung in der ersten und zweiten Drehrichtungsscheibe 524, 525 zu liegen kommt. Der zweite Kronenzapfen 528 ragt auf der, dem Schaftteil 52a gegenüberliegenden Seite aus dem Gegenkontakt teil 52b heraus. Die Stellwelle 52 ist wirkverbunden mit dem von der Stellwelle unabhängigen Gegenkontaktteil 52b.

Durch die Rotation und die Linearbewegung der bewegbar gelagerten Stellwelle 52 können verschiedene Kontakte auf einer Kontaktplatte 51 der Bedienungsmechanik 5 ausgelöst werden, von welchen elektronische Signale an die Elektronik 4 gesandt werden, wodurch die elektronische Steuerung des Multifunktionsgerätes 1 bewerkstelligt wird.

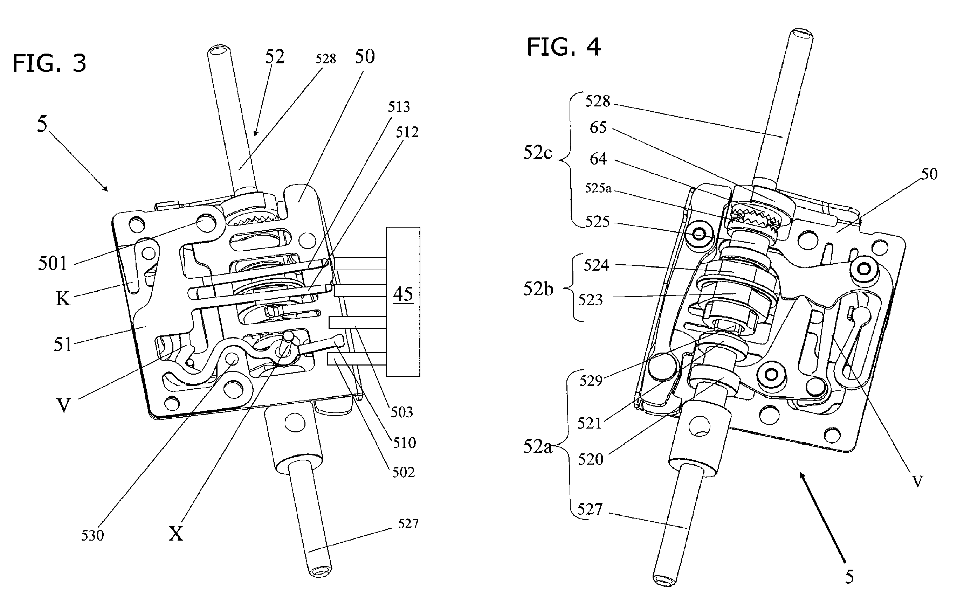

Die Basisplatte 50 und die Kontaktplatte 51 sind mit mindestens einem Bohrloch 501 versehen und werden mit Befestigungsmitteln 504 im Gehäuse 2 unlösbar befestigt. In Figur 3 ist eine Leiterplatte 45 schematisch angedeutet, welche elektrische Signale an die Elektronik 4 weiterleitet und elektrisch isoliert von der Basisplatte 50 angeordnet ist. Eine Kontaktzunge K der Kontaktplatte 51 ist immer mit der Leiterplatte 45 elektrisch leitend verbunden. Ein vorderer Linearkontakt 502 und ein hinterer Linearkontakt 503 werden je nach Richtung einer linearen Verschiebung der Stellwelle 52 ausgelöst. Die lineare Verschiebung der Stellwelle 52 führt zu einer Auslenkung eines Stellhebels 53, welcher um eine Stellhebelachse 530 verschwenkt wird, wobei eine Stellhebelfeder V gespannt wird. Ein Stellhebelstift X, welcher mit dem Stellhebel 53 verbunden ist, lenkt einen Linearbewegungskontakt 510 auf der Kontaktplatte 51 aus, welcher wiederum den vorderen oder den hinteren Linearkontakt 502, 503 kontaktiert. Damit werden die Linearkontakte 502, 503 durch die lineare Bewegung der Stellwelle 52 je nach Bewegungsrichtung ausgelöst, wodurch entsprechende Signale zur Kennzeichnung der Bewegungsrichtung an die Elektronik 4 weitergeben werden.

Wird der Schaftteil 52a der Stellwelle 52 in Richtung der Längsachse und der Drehachse der Stellwelle 52 gedrückt, dann wird der Linearbewegungskontakt 510 zwischen der ersten Scheibe 520 und der zweiten Scheibe 521 des Schaftteils 52a liegend, gegen den hinteren Linearkontakt 503 gedrückt und ein so genanntes "Push"-Signal wird an die Elektronik 4 gesendet, während die Stellwelle 52 in der Drückposition ist. Wird der Schaftteil 52a der Stellwelle 52 gezogen, dann berührt der Linearbewegungskontakt 510 den vorderen Linearkontakt 502 und ein so genanntes "Pull"-Signal wird an die Elektronik 4 gesendet, während die Stellwelle 52 in der Ziehposition ist.

In einer weiteren Ausführungsform kann die Elektronik 4 derart programmiert sein, dass einem Push-Signal bzw. einem Pull-Signal über einen längeren Zeitabschnitt, ein long-Push bzw. ein long-Pull Signal zugeordnet wird, was zu einem weiteren Manipulationsparameter führt, mit welchem Einstellungen des Multifunktionsgerätes 1 vornehmbar sind. Wenn also die Stellwelle 52 in einem Zeitraum von einigen hundert Millisekunden gedrückt bzw. gezogen gehalten wird, dann erkennt die Elektronik 4 dies als long-Push bzw. long-Pull Signal.

Zur Detektion der Drehbewegung und der Drehrichtung weist die erfindungsgemässe Bedienungsmechanik 5 in einer möglichen Ausführungsform auf der Kontaktplatte 51 den ersten Drehrichtungskontakt 512 und den zweiten Drehrichtungskontakt 513 auf, welche zungenförmig ausgestaltet sind. Die erste Drehrichtungsscheibe 523 und die zweite Drehrichtungsscheibe 524 am Gegenkontaktteil 52b arbeiten mit dem ersten und zweiten Drehrichtungskontakt 512, 513 zusammen. Beide Drehrichtungsscheiben 523, 524 sind identisch geformt und weisen jeweils abwechselnd entlang des äusseren Scheibenumfangs plane ebene Flächenabschnitte und konvexe Flächenabschnitte äquivalent einer Kurvensteuerungsscheibe auf. Durch die Rotation der Stellwelle 52 drücken die Drehrichtungsscheiben 523, 524 die Drehrichtungskontakte 512, 513 entsprechend nach oben gegen die Leiterplatte 45, wodurch elektrische Signale an die Elektronik 4 gesendet werden. Neben der Erzeugung von elektrischen Signalen durch die mechanische Verdrehung der Drehrichtungsscheiben 523, 524 sind auch andere Auslösemechanismen von elektrischen Signalen in der Elektronik 4 vorstellbar.

Da die erste Drehrichtungsscheibe 523 am Gegenkontaktteil 52b gerade so relativ zur zweiten Drehrichtungsscheibe 524 befestigt ist, dass jeweils immer nur ein Drehrichtungskontakt 512, 513 zur Zeit im elektrischen Kontakt mit der korrespondierenden Drehrichtungsscheibe ist, kann die Elektronik 4 die Drehrichtung der Stellwelle 52 detektieren, so dass auf der Anzeigeeinheit 40 die physische Bewegung der Stellwelle 52 zur Bewegung durch Menüs 10 und Untermenüs 11 eingesetzt werden kann. Je nach Rotationsrichtung der Stellwelle 52 wird so ein aufwärts Scrollen oder ein abwärts Scrollen durch den Benutzer in Menüs und Untermenüs ermöglicht.

Die hier offenbarte Stellwelle 52 weist einen Gegenkontaktteil 52b auf, welcher rotativ beweglich aber linear unbeweglich relativ zur Basisplatte 50 und zur Kontaktplatte 51 ausgeführt ist, so dass die elektrischen Kontakte 512, 513 die korrespondierenden Drehrichtungsscheiben 523, 524 zu jeder Zeit erreichen können. Dadurch bleibt der Gegenkontaktteil 52b zu jeder Zeit mit mindestens einem ersten Drehrichtungskontakt 512, sowie mit mindestens einem zweiten Drehrichtungskontakt 513 in Kontakt. Die lineare Unbeweglichkeit kann mit einem Gegenkontaktteillager erreicht werden, welches die Bewegung relativ zur Bedienungsmechanik 5 verhindert.

Sobald die Stellwelle 52 mittels der ersten Bedienkrone 56 gedrückt wird und damit ein Push-Signal oder ein long-Push-Signal ausgeführt und von der Elektronik 4 detektiert wird, kann das Multifunktionsgerät 1 gesteuert werden. Ebenso kann ein Pull-Signal bzw. ein long-Pull-Signal eingesetzt werden, um das Multifunktionsgerät 1 zu manipulieren. Die hier vorgestellte erste Ausführungsform eines Multifunktionsgerätes 1 umfasst damit eine Bedienungsmechanik 5 mit nur einem Bedienelemente, einer Stellewelle 52, durch welche ein Push- und ein long-Push-Signal, ein Pull- und ein long-Pull-Signal und zwei Scroll-Signale an die Elektronik 4 weitergegeben werden können, wobei die Stellwelle 52 von einer Neutralposition in eine Drückposition und in eine Ziehposition bewegbar ist.

Eine vorgesehene Rückfederung der Stellwelle 52, welche durch die Rückfederung der Stellhebelfeder V gegen den Stellhebel 53 wirkt oder die federnde zungenförmige Ausgestaltung des Linearbewegungskontaktes 510 erreicht werden kann, führt dazu, dass sich die Stellwelle 52 nach der linearen Auslenkung bei fehlendem Druck oder Zug zurück in die Neutralposition bewegt, damit durch die Rotation der Stellwelle 52 durch ein Menü 10 gescrollt werden kann und verschiedene Parameter einstellbar sind. Die Rotation der Stellwelle erfolgt bei der Bedienung des Multifunktionsgerätes 1 ohne linearen Druck oder Zug, so dass bei der Rotation der Stellwelle 52 nur die Drehrichtungskontakte 512, 513 zur Zeit aktiviert werden.

Durch eine entsprechend grosse Ausführung der ersten Bedienkrone 56 kann ein Benutzer die Stellwelle 52 der Bedienungsmechanik 5 auch mit schweissnassen Händen oder mit Handschuhen an den Händen benutzen und eine gewünschte Anzeige einstellen.

In einer weiteren Ausführungsform der Bedienungsmechanik 5 ist die Stellwelle 52, das Gehäuse 2 vollständig querend und damit wirkungsmässig durch das Gehäuse 2 durchgehend, geführt, wobei der zweite Kronenzapfen 528 das Gehäuse 2 verlässt. Auf den aus dem Gehäuse 2 austretenden zweiten Kronenzapfen 528 wird eine zweite Bedienkrone 57 kraft- und/oder formschlüssig befestigt, welche wie die erste Bedienkrone 56 Aufrauhung oder Griffigkeit erhöhende Mittel, wie eine Riffelung aufweist, wodurch die Griffigkeit erhöht ist und die Bedienerfreundlichkeit gesteigert ist. Die, das Gehäuse 2 vollständig querende Stellwelle 52 wird in einem zweiten Lager 60 rotativ und linear bewegbar gehalten, wodurch eine höhere Stabilität erreicht wird.

Die Bewegung der Stellwelle 52 kann in dieser Ausführungsform wahlweise mit der ersten oder der zweiten Bedienkrone 56, 57 ausgeführt werden, wobei die Druckposition der Stellwelle 52 durch eine lineare Drückbewegung der ersten Bedienkrone 56 in Richtung des Gehäuses 2, oder eine Zugbewegung der zweiten Bedienkrone 57 weg vom Gehäuse 2 erfolgen kann, was jeweils zu einem Push-Signal bzw. zu einem long-Push-Signal an die Elektronik 4 führt. Entsprechend verhält es sich mit der Ziehposition und damit der Erzeugung eines Pull-Signals bzw. eines long-Pull-Signals.

Durch die Möglichkeit der Bedienung der Stellwelle 52 von zwei diametral entgegengesetzten Seiten des Gehäuses 2 ist die vorliegende Bedienungsmechanik 5 von Linkshändern und Rechtshändern gleichermassen gut bedienbar.

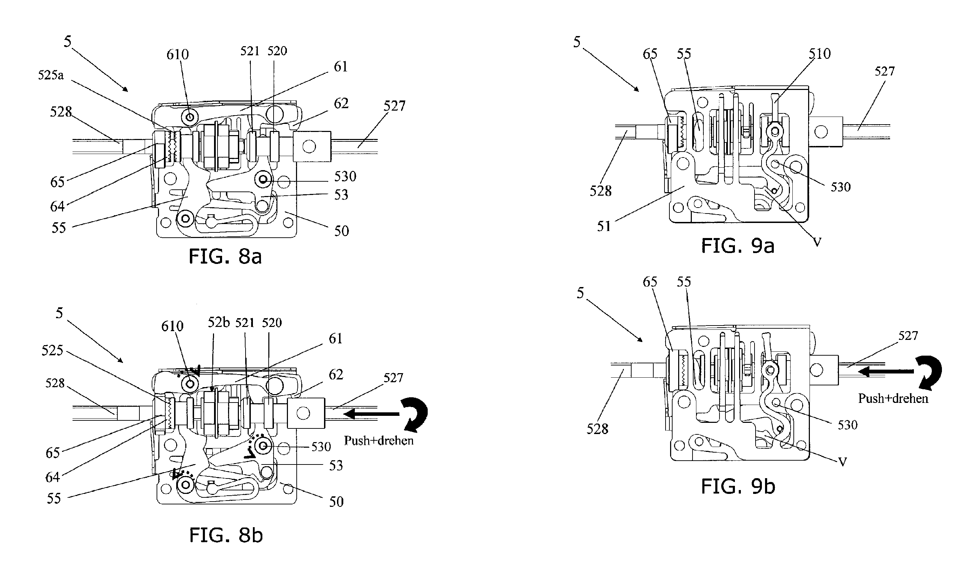

Die erfindungsgemässe Bedienmechanik 5 ist mit einer Arretiermechanik versehen, welche unabhängig davon ob, die Bedienmechanik mit nur einer ersten Bedienkrone 56, oder mit einer ersten Bedienkrone 56 und einer zweiten Bedienkrone 57 versehen ist, benutzbar ist. Diese Arretiermechanik dient dazu eine ungewollte Betätigung der Stellwelle 52 zu verhindern. Eine mögliche Ausführungsform einer Arretiermechanik ist in den Figuren 8a und 8b dargestellt, wobei Figur 8a eine Ansicht der Bedienungsmechanik 5 auf die Basisplatte 50 zeigt, wobei sich die Stellwelle 52 in der unausgelenkten Neutralposition befindet, in welcher keine Auslenkung des Linearbewegungskontaktes 510 vorliegt.

Um das elektronische Multifunktionsgerät 1 gegen eine Betätigung der Stellwelle 52 und damit gegen eine ungewollte Eingabe von Signalen zu sichern, ist der Kupplungsteil 52c im Bereich nach dem Steg 529 nach Durchgang durch den Gegenkontaktteil 52 vorgesehen. Der Kupplungstrieb 525, umfassend das erste Zahnrad 525a ist auf den Steg 529, mit rechteckigem Querschnitt mit der Stellwelle 52 mitbewegbar, aufgesteckt. Der Kupplungstrieb 525 macht die lineare Verschiebung und die Rotationsbewegungen der Stellwelle 52 mit, da der Steg 529 den Kupplungstrieb 525 entsprechend mitnimmt. Der zweite Kronenzapfen 528 mit kreisförmigem Querschnitt ragt durch eine Bohrung in der Profilscheibe 65, welche ebenfalls einen kreisförmigen Querschnitt aufweist. Mittels eines Scheibenlagers 66 ist die Profilscheibe 65 auf der Basisplatte 50 linear unbewegbar gelagert, so dass der zweite Kronenzapfen 528 durch die Profilscheibe 65 ein- und ausfahren kann.

Das an der Profilscheibe 65 angeformte zweite Zahnrad 64 nimmt das erste Zahnrad 525a auf, wenn die Stellwelle 52 von der Bedienkrone 56 in Richtung Gehäuse 2 gedrückt wird, also eine push-Bewegung, oder wenn die Stellwelle 52 mit der zweiten Bedienkrone 57 aus dem Gehäuse 2 gezogen wird, also eine pull-Bewegung ausgeführt wird.

In der hier beschriebenen Ausführungsform einer Arretiermechanik ist ein Stellhebel 53, eine Arretierwippe 55 und ein Verriegelungshebel 61 vorgesehen, welche auf der Basisplatte 50 zusammenwirkend schwenkbar befestigt sind, vorgesehen. Wenn die Stellwelle 52 gedrückt wird, wird der Stellhebel 53 um eine Stellhebelachse 530 verschwenkt. Durch den Stellhebel 53 wird die der Stellwelle 52 zugewandte Seite der Arretierwippe 55 in Richtung der push-Bewegung geführt, wodurch eine Federspannung in die der push-Bewegung entgegengesetzte Richtung entsteht. Das erste Zahnrad 525a greift bei diesem Vorgang in das zweite Zahnrad 64 der Profilscheibe 65 in.

Wird während der push-Bewegung eine Rotation der Stellwelle 52 durchgeführt, dann wird die Profilscheibe 65 rotiert, da das erste Zahnrad 525a in das zweite Zahnrad 64 eingreift. Bei der Rotation der Profilscheiben 65 lenkt der äussere Umfang der Profilscheibe 65 den schwenkbewegbar gelagerten Verriegelungshebel 61 aus, so dass der Verriegelungshebel 61 um eine Verriegelungshebelachse 610 in Richtung der Stellwelle 52 geschwenkt wird.

Ein am Verriegelungshebel 61 schwenkbar befestigter Doppelriegel 62 greift zwischen die erste Scheibe 520 und die zweite Scheibe 521 ein, wodurch die lineare Verschiebung der Stellwelle 52 unterbunden wird und keine weiteren push- oder pull-Signale mehr gesendet werden können. Die bei der weiteren Rotation der Stellwelle 52 auftretenden Signale werden nicht von der Elektronik 4 verarbeitet, da das push-Signal zeitgleich ausgelöst wird.

In der hier gewählten Ausführungsform weist die Profilscheiben 65 eine Profilierung auf, welche einen kleinen Kreissektor mit einem kleinen Kreisbogen und einen grossen Kreissektor mit einem grossen Kreisbogen umfasst. Dies ist in Figur 11a detailliert gezeigt. Der äussere Umfang der Profilscheibe 65 betätigt den Verriegelungshebel 61 nur dann, wenn der grosse Kreisbogen am Verriegelungshebel 61 anliegt. Wird die Stellwelle 52 zurückgedreht oder weitergedreht, so dass der kleine Kreisbogen dem Verriegelungshebel 61 gegenüberliegt, so hat die Profilscheibe 65 keinen Kontakt mehr zum Verriegelungshebel 61, womit die Arretierung der Stellwelle 52 durch das zurückschwenken des Verriegelungshebels 61 aufgehoben wird. Zur Arretierung der Stellwelle 52 kann die Drehbewegung im Uhrzeigersinn oder gegen den Uhrzeigersinn erfolgen. Die hier beschriebene Art des Arretiermechanismus erlaubt die Drehung in beide möglichen Richtungen.

Bei einer Ausgestaltung der Bedienungsmechanik 5 mit einer ersten Bedienkrone 56 und einer zweiten Bedienkrone 57 ist die Betätigung der Arretiermechanik mit der ersten Bedienkrone 56 oder der zweiten Bedienkrone 57 vorgesehen, dabei entspricht die push-Bewegung der ersten Bedienkrone 56 der pull-Bewegung der zweiten Bedienkrone 57.

Das Eingreifen des ersten Zahnrades 525a in das zweite Zahnrad 64 ist in jeder Winkelstellung der Stellwelle 52 möglich, so dass die Stellwelle jederzeit durch Drücken der Stellwelle 52 in das Gehäuse bzw. durch die entsprechende pull-Bewegung der Stellwelle 52 mit der zweiten Bedienkrone 57 bei gleichzeitiger Drehbewegung der Stellwelle 52 durchführbar ist. Diese Möglichkeit der Arretierung ist wesentlich einfacher und bedienerfreundlicher als der Arretiermechanismus von Armbanduhren.

Eine Arretierung der Stellwelle 52 ist ebenfalls möglich durch das Herausziehen des zweiten Kronenzapfens 528 aus dem Gehäuse 2, was dem Hineindrücken des Schaftteils 52a in das Gehäuse entspricht. Da die Stellwelle 52 über der Bedienmechanik 5 schwebend gelagert ist, ist es möglich, die Stellwelle 52 durch das Hineindrücken des Kupplungsteils 52c in das Gehäuse 2 zu arretieren, wobei das zweite Zahnrad 64 in das erste Zahnrad 525a eingreift und die Arretierung wie oben beschrieben ausführbar ist.

### Bezugszeichenliste

- 1: elektronisches Multifunktionsgerät
- 2: Gehäuse
20 Gehäuserand
- 3: Armband
- 4: Elektronik
40 Anzeigeeinheit
41 Sensoreinheit
(Drucksensor, Lagesensor, Magnetfeldsensor, UV-Sensor, RF-Interface mit Antenne)
42 erster Mikroprozessor
43 Schnittstelle
44 zweiter optionaler Mikroprozessor
45 Leiterplatte
- 5: Bedienungsmechanik
50 Basisplatte
501 Bohrlöcher zur Befestigung der Basisplatte im/am Gehäuse
502 vorderer Linearkontakt
503 hinterer Linearkontakt
504 Befestigungsmittel
51 Kontaktplatte
510 Linearbewegungskontakt
512 erster Drehrichtungskontakt
513 zweiter Drehrichtungskontakt
- 52: Stellwelle (Bedienelement)
52a Schaftteil
520 erste Scheibe
521 zweite Scheibe
527 erster Kronenzapfen
527a Gewinde
528 zweiter Kronenzapfen 528a Gewinde
529 Steg

- 52b: Gegenkontaktteil
523 erste Drehrichtungsscheibe
524 zweite Drehrichtungsscheibe
- 52c: Kupplungsteil
525 Kupplungstrieb
525a erstes Zahnrad
- 53: Stellhebel
530 Stellhebelachse
- 55: Arretierwippe
- 56: erste Bedienkrone
- 57: zweite Bedienkrone

- 59: erstes Lager
- 60: zweites Lager
- 61: Verriegelungshebel
610 Verriegelungshebelachse
- 62: Doppelriegel
- 64: zweites Zahnrad
- 65: Profilscheibe
- V: Stellhebelfeder
- X: Stellhebelstift
- K: Kontaktzunge

- 10: Menü
- 11: Untermenü
- 12: kleiner Kreisbogen
- 13: grosser Kreisbogen

## Patentansprüche

1. Bedienungsmechanik (5) eines am Handgelenk einer Person tragbaren elektronischen Multifunktionsgerätes (1), insbesondere eines Pulsmonitors, eines Abspielgerätes für Multimediaanwendungen oder eines Kommunikationsgerätes, wobei das Multifunktionsgerät (1)
ein Gehäuse (2) mit einem Gehäuserand (20) aufweist, welches mittels eines Armbandes (3) am Handgelenk lösbar befestigbar ist, wobei innerhalb des Gehäuses (2)
die Bedienmechanik (5) und
eine integrierte Elektronik (4) angeordnet ist, welche durch eine Mehrzahl elektronischer Kontakte (510, 512, 513) steuerbar ist und mindestens eine mehrzeilige Anzeigeeinheit (40) und eine Sensoreinheit (41) mit einer Mehrzahl von Sensoren für verschiedene Messungen umfasst, wobei mindestens ein erster Mikroprozessor (42) die Datenverarbeitung, die Anzeige von Werten auf der Anzeigeeinheit (40) und die Speicherung von Werten steuert,
**dadurch gekennzeichnet, dass** die Bedienmechanik (5) mit mindestens einem Bedienelement (52) in Form einer Stellwelle (52) wirkverbunden ist, wobei
die Stellwelle (52)
innerhalb der Bedienungsmechanik (5) im Gehäuse (2),
das Gehäuse (2) teilweise querend parallel zu einer flächigen Ausdehnung des Gehäuses (2), angeordnet ist und
rotativ um eine Drehachse und linear parallel zur Drehachse der Stellwelle (52) bewegbar gelagert ist, wobei ein,
aus dem Gehäuse (2) herausragender erster Kronenzapfen (527) der Stellwelle (52) mit einer ersten Bedienkrone (56) verbunden ist und somit von einem Benutzer mit den Fingerspitzen linear und rotativ bewegbar ist, wodurch die Mehrzahl elektronischer Kontakte (510, 512, 513) entsprechend der Bewegungsrichtungen der Stellwelle (52) auslösbar ist, woraus Signale zur Steuerung der Elektronik (4) des Multifunktionsgerätes (1) resultieren und der Benutzer durch Menüs (10) und Untermenüs (11) scrollen kann.

2. Bedienungsmechanik nach Anspruch 1, **dadurch gekennzeichnet, dass** die Stellwelle (52) einen Schaftteil (52a), umfassend den ersten Kronenzapfen (527), einen Steg (529), eine erste Scheibe (520) und eine zweite Scheibe (521) aufweist.

3. Bedienungsmechanik nach Anspruch 2, **dadurch gekennzeichnet, dass** die Stellwelle (52) mit einem Gegenkontaktteil (52b) der Bedienungsmechanik (5) zusammenwirkt, welcher rotativ beweglich, aber linear unbeweglich ist und zu jeder Zeit in Kontakt mit mindestens einem ersten Drehrichtungskontakt (512), sowie mit mindestens einem zweiten Drehrichtungskontakt (513) ist.

4. Bedienungsmechanik nach Anspruch 1, **dadurch gekennzeichnet, dass** die Stellwelle (52) der Bedienungsmechanik (5) eine Neutralposition aufweist, in welcher in welcher keine Auslenkung des Linearbewegungskontaktes 510 vorliegt.

5. Bedienungsmechanik nach Anspruch 1, **dadurch gekennzeichnet, dass** die Stellwelle (52) der Bedienungsmechanik (5) eine Druckposition aufweist, in welcher die Stellwelle (52) von der Seite des Schaftteils (52a) mit der ersten Bedienkrone (56) in Richtung linear parallel zur Drehachse der Stellwelle (52) in Richtung Gehäuse (2) gedrückt ist.

6. Bedienungsmechanik nach Anspruch 1, **dadurch gekennzeichnet, dass** die Stellwelle (52) der Bedienungsmechanik (5) eine Ziehposition aufweist, in welcher die Stellwelle (52) von der Seite der ersten Bedienkrone (56) in Richtung linear parallel zur Drehachse der Stellwelle (52) vom Gehäuse (2) weg gezogen ist.

7. Bedienungsmechanik nach Anspruch 1, **dadurch gekennzeichnet, dass** die Stellwelle (52)
durch das Gehäuse (2), das Gehäuse (2) vollständig querend und damit wirkungsmässig durchgehend durch das Gehäuse (2) geführt ist.

8. Bedienungsmechanik nach Anspruch 1, **dadurch gekennzeichnet, dass** eine zweite Bedienkrone (57), der ersten Bedienkrone (56) diametral dem Schaftteil (52a) gegenüberliegend aus dem Gehäuse (2) herausragend, an einem zweiten Kronenzapfen (528) der Stellwelle (52) form- und/oder kraftschlüssig lösbar verbunden angeordnet ist.

9. Bedienungsmechanik nach Anspruch 8, **dadurch gekennzeichnet, dass** die Stellwelle (52) von beiden diametral gegenüberliegenden Seiten des Gehäuses (2) bedienbar ist.

10. Bedienungsmechanik nach Anspruch 8, **dadurch gekennzeichnet, dass** das Drücken des Schaftteils (52a) in Richtung Gehäuse (2) einem Ziehen der Stellwelle (52) an der zweiten Bedienkrone (57) aus dem Gehäuse (2) heraus entspricht.

11. Bedienungsmechanik nach Anspruch 8, **dadurch gekennzeichnet, dass** die erste Bedienkrone (56) und/oder die zweite Bedienkrone (57) eine Aussenverzahnung aufweisen, so dass die erste Bedienkrone (56) und/oder die zweite Bedienkrone (57) mit schweissnassen Händen, sowie mit handschuhtragenden Händen bedienbar ist.

12. Bedienungsmechanik nach Anspruch 10, **dadurch gekennzeichnet, dass** die Stellwelle (52) mittels eines Verriegelungshebels (61), welcher mit einer Profilscheibe (65), die rotativ bewegbar auf der Basisplatte (50) gelagert ist, gegen eine ungewollte Betätigung sicherbar ist.

13. Bedienungsmechanik nach Anspruch 11, **dadurch gekennzeichnet, dass** die Verriegelung durch Drücken von der Seite des Schaftteils (52a) in Richtung Gehäuse (2) oder Ziehen des Kupplungsteils (52c aus dem Gehäuse (2) heraus bei gleichzeitiger Rotation der Stellwelle (52) erreichbar ist.
